**Europäisches Patentamt**

**European Patent Office**

**Office européen des brevets**

(11) Veröffentlichungsnummer: **0 342 435**

**A1**

## (12) EUROPÄISCHE PATENTANMELDUNG

(21) Anmeldenummer: **89108032.7**

(22) Anmeldetag: **03.05.89**

(51) Int. Cl.⁴: **A61M 16/00**

(30) Priorität: **19.05.88 DE 3817091**

(43) Veröffentlichungstag der Anmeldung:
**23.11.89 Patentblatt 89/47**

(84) Benannte Vertragsstaaten:
**DE FR GB SE**

(71) Anmelder: **Drägerwerk Aktiengesellschaft**
**Moislinger Allee 53-55**
**D-2400 Lübeck 1(DE)**

(72) Erfinder: **Kiske, Siegfried, Dr. Dipl.-Ing.**
**Am Sonnenberg 1e**
**D-2401 Gross Grönau(DE)**
Erfinder: **Schwanbom, Erik, Dr. Dipl.-Chem.**
**Claudiusring 38a**
**D-2400 Lübeck(DE)**
Erfinder: **Wallroth, Carl Friedrich, Dr. Dipl.-Ing.**
**Stresemannstrasse 1**
**D-2400 Lübeck(DE)**

(54) **Kolben-Zylindereinheit als Fördervorrichtung in einem Beatmungsgerät.**

(57) Eine Kolben-Zylindereinheit (1, 4) als eine durch eine Antriebsvorrichtung (7) betätigbare Fördereinheit für das Atemgas im Atemkreislauf eines Beatmungsgerätes, bei welcher der Kolben (4) gegenüber dem Zylinder (1) durch ein symmetrisch zueinander angeordnetes Rollmembranpaar (8,9) abgedichtet ist, welches den Zylinderinhalt in eine Vorkammer (18) und eine Arbeitskammer (20) unterteilt, soll derart verbessert werden, daß der Verschleiß der Rollmembranen (8,9) während der Arbeitshübe und insbesondere beim Start durch ein sauberes Abrollen der Falten verringert und eine Dichtheitskontrolle auch während des Betriebes sowie ein unverzügliches Erkennen von durch Biegebeanspruchung, Alterung oder sonstige Einflüsse eintretenden Leckagen ermöglicht wird. Dazu ist vorgesehen, daß der von den Rollmembranen (8, 9) gemeinsam eingeschlossene Innenraum (15) unter einem solchen Innendruck abweichend von dem Druck sowohl in der Vorkammer (18) als auch der Arbeitskammer (20) gehalten ist, daß der höhere der Drücke auf den konkaven Innenseiten der Falten (13, 14) ansteht.

## Kolben-Zylindereinheit als Fördervorrichtung in einem Beatmungsgerät

Die Erfindung betrifft eine Kolben-Zylindereinheit als eine durch eine Antriebsvorrichtung betätigbare Fördereinheit für das Atemgas im Atemkreislauf eines Beatmungsgerätes mit den Merkmalen des Oberbegriffs des Anspruchs 1.

Zur Förderung des Atemgases im Atemkreislauf eines Beatmungsgerätes oder Narkosebeatmungsgerätes werden Antriebseinheiten in unterschiedlicher Ausführungsform benutzt, von denen eine bekannte Antriebsart in der Monographie: "Automatic Ventilation of the lungs", William W. Mushin et al., Blackwell Scientific Publications, 3. Auflage, Seite 273 bis Seite 277 beschrieben ist. Dort ist ein Beatmungsgerät schematisch dargestellt, mit welchem das Atemgas durch eine Kolben-Zylindereinheit in den Atemkreislauf gefördert wird. Die Außenfläche des Kolbens ist gegenüber der Innenwand des Zylinders durch zwei Rollmembranen abgedichtet, welche bei einer Kolbenbewegung in ihrer zwischen dem Kolben und dem Zylinder angeordneten Falte der Hubbewegung in beiden Richtungen folgen können. Die Rollmembranen teilen den Zylinderinnenraum einerseits in eine Arbeitskammer zur Erzeugung des für die Beatmung notwendigen Überdruckes und andererseits in eine Vorkammer, in welche während des Arbeitshubes diejenige Luft angesaugt wird, welche zur Erzeugung eines Unterdruckes während der Exspiration dient. Als Antriebsvorrichtung wird ein Elektromotor mit einem Getriebe und einem Hebelgestänge zur Übersetzung der Drehbewegung in eine Hubbewegung eingesetzt. Während der Beatmung führt der Kolben abwechselnd Hubbewegungen zur Erzeugung von Inspirations- und Exspirationsphasen aus, welche in ihrer Frequenz und Hublänge über das Getriebe und das Hubgestänge variiert werden können. Je nach Ansteuerung des Kolbens üben die Rollmembranen demnach einen mehr oder weniger langen Hubweg aus, während dem die entsprechende Falte der Rollmembran in dem Zwischenraum zwischen Kolben und Zylinderinnenfläche dem Hubweg folgend abrollt.

Bei einer Anordnung der Rollmembranen nach der bekannten Kolben-Zylindereinheit ist es von Nachteil, daß durch die wechselnden Druckverhältnisse sowohl in der Vorkammer als auch in der Arbeitskammer die Abrollfähigkeit des Membranmaterials in der näheren Umgebung der Falte ungleichmäßig und unvorhersehbar wird und sogar versagen kann, wenn das Membranmaterial in der Falte aneinander hängenbleibt. Insbesondere ist diese Gefahr beim Starten des Kolbens gegeben, weil sich dann noch kein Beatmungsdruck aufgebaut hat. Ein derartiger nicht bemerkbarer Verschleiß führt leicht zu Undichtheiten des Materials, somit zu Druckeinbrüchen und Gasverlusten, welche bei den heutigen, knapp dosierten Gasmengen bei Beatmungsformen mit immer geringerem Gasüberschuß und praktisch geschlossenem Atemkreislauf zu ernsthaften Folgen führen können.

Der vorliegenden Erfindung liegt somit die Aufgabe zugrunde, eine Kolben-Zylindereinheit der genannten Art so zu verbessern, daß der Verschleiß der Rollmembranen während der Arbeitshübe und insbesondere beim Start durch ein sauberes Abrollen der Falten verringert und eine Dichtheitskontrolle auch während des Betriebes sowie ein unverzügliches Erkennen von durch Biegebeanspruchung, Alterung oder sonstige Einflüsse eintretenden Leckagen ermöglicht wird.

Die Lösung der Aufgabe erfolgt durch die im kennzeichnenden Teil des Anspruchs 1 angeführten Merkmale.

Durch die Druckversorgung des von den Rollmembranen gemeinsam eingeschlossenen Innenraumes zwischen dem Kolben und dem Zylinder wird für eine während des gesamten Atemhubs gleichbleibende Ausbildung der Falte und ihr glattes Anliegen an den Wänden von Kolben und Zylinder gesorgt, und damit deren reibungs- und verschleißfreie Bewegung ermöglicht. Durch das am gesamten Umfang des Kolbens stattfindende gleichmäßige Abwälzen der Rollmembran wird eine einseitige Belastung vermieden. Mittels Überwachung des Innenraumdruckes ist eine Dichtheitskontrolle während des Betriebes einfach ausführbar und das Auftreten von Leckagen unverzüglich zu erkennen.

Für den Fall, daß die konvexen Außenseiten der Falten einander zugekehrt sind, wird der von ihnen eingeschlossene Innenraum auf einem Unterdruck gehalten.

Sind hingegen die konkaven Innenseiten der Falten einander zugekehrt, ist der Innenraum auf einem Überdruck zu halten.

Durch den im Innenraum vorherrschenden Druck wird gewissermaßen eine Stabilisierung der Materialflächen der Rollmembranen erreicht, welche eine exakte axiale Hubbewegung ermöglichen. Damit ist es möglich geworden, die Hubbewegung des Kolbens selbst durch einen Wegaufnehmer festzustellen. Es ist auf einfache Weise ein Bezug zwischen zurückgelegtem Kolbenhubweg und gefördertem Atemgasvolumen herstellbar. Da die Wandungen der Rollmembranen nunmehr einem in der Vorkammer bzw. Arbeitskammer herrschenden, vom Innenraumdruck abweichenden Druck nicht mehr nachgeben, sind die Umrechnungen von Hubweg zu Hubvolumen mit einem solchen geringen Fehler behaftet, daß auch geringe Atemgasvolumina mit einer Kolben-Zylindereinheit dosierbar werden.

Zweckmäßigerweise wird der von den Rollmembranen eingeschlossene Innenraum an eine Druckversorgungseinheit und an eine den Innendruck aufnehmende Druckmeßeinrichtung angeschlossen. Somit kann zum einen der Innendruck je nach Betriebsbedingungen und Erfordernissen eingestellt, bzw. der im Innenraum vorherrschende Druck überwacht werden. Damit sind auch Dichtheitskontrollen während des Betriebes der Kolben-Zylindereinheit jederzeit möglich, und eventuell auftretende Leckagen infolge von Biegebeanspruchung, Alterung oder sonstigen Einflüssen können unverzüglich erkannt werden.

Es ist zweckmäßig, die Druckmeßeinrichtung über eine Signalleitung an die Versorgungseinheit anzuschließen, damit ihr gemessener Druckistwert mit dem in der Versorgungseinheit speicherbaren Drucksollwert verglichen werden kann. Auf diese Weise können auch variabel einstellbare Druckwerte für den Innenraum überwacht werden.

Bei fehlender Übereinstimmung zwischen Druckistwert und Drucksollwert ist es zweckmäßig, die Antriebsvorrichtung durch eine Steuereinheit auszuschalten und dadurch ein weiteres fehlerhaftes Betreiben der Kolben-Zylindereinheit zu verhindern. Durch zusätzliches Warnen bei derart unerwünschtem fehlerhaften Betriebszustand wird ein unmittelbares Beseitigen des Fehlers durch das bedienende Personal ermöglicht.

Ein Ausführungsbeispiel der Erfindung wird anhand der schematischen Zeichnung dargestellt und im folgenden näher erläutert.

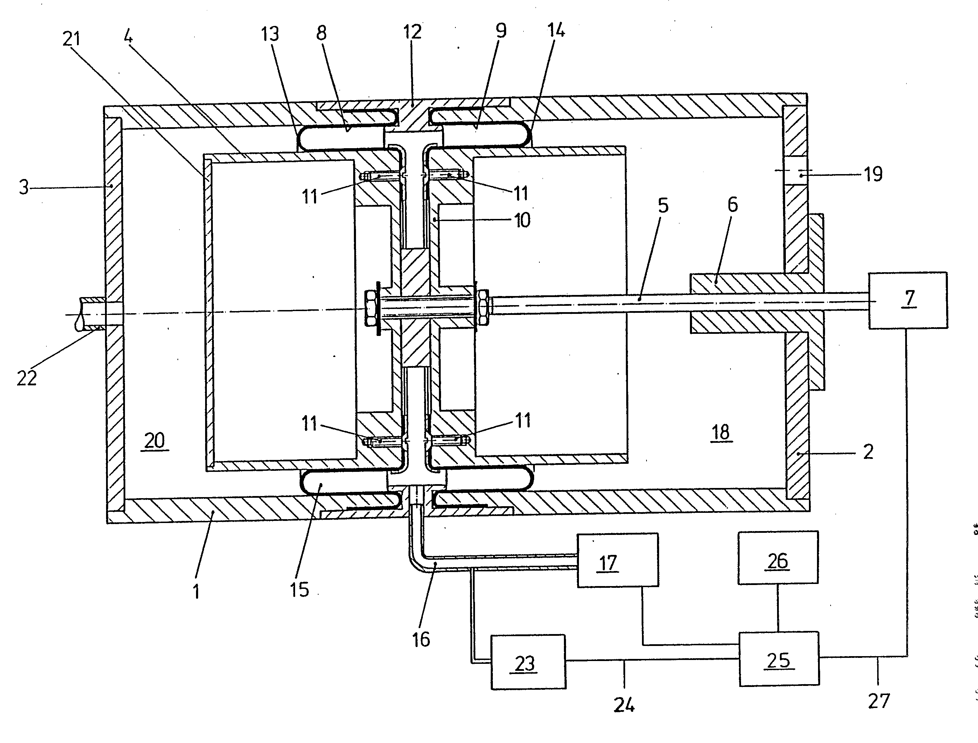

In der einzigen Figur ist ein Zylinder (1) dargestellt, dessen Stirnseiten durch eine Grundplatte (2) und eine Anschlußplatte (3) abgeschlossen sind. Im Innenraum des Zylinders (1) ist ein Kolben (4) auf einer in einem Axiallager (6) geführten Kolbenstange (5) aufgenommen, die mit einer Antriebsvorrichtung (7) verbunden ist.

Die Außenfläche des Kolbens (4) ist gegenüber dem Zylinder (1) mit einer Doppelrollmembran (8, 9) abgedichtet, welche einerseits an einem radial innerhalb des Kolbens (4) verlaufenden Zwischenboden (10) mittels Schrauben (11) befestigt, und andererseits im Zylinder (1) über eine Klemmverbindung (12) eingespannt ist. Die konkaven Innenseiten der Falten (13, 14) sind einander zugekehrt, wobei der von den Membranen (8, 9) eingeschlossene Innenraum (15) über eine Druckleitung (16) durch eine Druckversorgungseinheit (17) auf einen Überdruck gehalten ist. Die Membranen (8, 9) teilen den Innenraum des Zylinders (1) in eine Vorkammer (18), welche über einen Durchlaß (19) mit der Umgebung verbunden ist, und in eine Arbeitskammer (20), in welcher der durch die Hubbewegung des Kolbens (4) erzeugte Arbeitsdruck herrscht. Der Kolben (4) besitzt an seiner in der Arbeitskammer befindlichen Stirnseite eine Stirnplatte (21). Das während des Arbeitshubes verdrängte Atemgas kann durch den an der Anschlußplatte (3) angebrachten Anschlußstutzen (22) zu einem nicht dargestellten Atemkreislauf eines ebenfalls nicht dargestellten Beatmungsgerätes gefördert werden. An die Druckleitung (16) ist eine Druckmeßeinrichtung (23) angeschlossen, deren Meßsignal über eine Signalleitung (24) an eine Steuereinheit (25) weitergeleitet wird, welche gleichzeitig den in der Druckversorgungseinheit (17) eingegebenen Drucksollwert aufnimmt. Eine Anzeigeeinheit (26) zeigt den aktuellen Zustand an und gibt eventuell notwendige Warnsignale ab. Im Falle eines fehlerhaften Betriebszustandes, wie er beispielsweise aus einer unerwünschten Abweichung zwischen Druckistwert und -sollwert herrühren kann, ist die Antriebsvorrichtung (7) durch ein entsprechendes Signal der Steuereinheit (25) über die Steuerleitung (27) abschaltbar.

## Ansprüche

1. Kolben-Zylindereinheit als eine durch eine Antriebsvorrichtung betätigbare Fördereinheit für das Atemgas im Atemkreislauf eines Beatmungsgerätes, bei welcher der Kolben gegenüber dem Zylinder durch ein symmetrisch zueinander angeordnetes Rollmembranpaar abgedichtet ist, welches den Zylinderinhalt in eine Vorkammer und eine Arbeitskammer unterteilt, dadurch gekennzeichnet, daß der von den Rollmembranen (8, 9) gemeinsam eingeschlossene Innenraum (15) unter einem solchen Innendruck abweichend von dem Druck sowohl in der Vorkammer (18) als auch der Arbeitskammer (20) gehalten ist, daß der höhere der Drücke auf den konkaven Innenseiten der Falten (13, 14) ansteht.

2. Kolben-Zylindereinheit nach Anspruch 1, dadurch gekennzeichnet, daß bei einander zugekehrten konvexen Außenseiten der Falten (13, 14) der Innenraum (15) auf einem Unterdruck gegenüber den Kammern (18, 20) gehalten ist.

3. Kolben-Zylindereinheit nach Anspruch (1), dadurch gekennzeichnet, daß bei einander zugekehrten konkaven Innenseiten der Falten (13, 14) der Innenraum (15) auf einem Überdruck gegenüber den Kammern (18, 20) gehalten ist.

4. Kolben-Zylindereinheit nach einem der Ansprüche 1-3, dadurch gekennzeichnet, daß an der Antriebseinheit (7) ein Wegaufnehmer zur Feststellung des vom Kolben (4) zurückgelegten Weges vorgesehen ist.

5. Kolben-Zylindereinheit nach einem der Ansprüche 1-4, dadurch gekennzeichnet, daß der von den Rollmembranen (8, 9) eingeschlossene Innenraum (15) an eine Druckversorgungseinheit (17) und an eine den Innendruck aufnehmende Druckmeßeinrichtung (23) angeschlossen ist.

6. Kolben-Zylindereinheit nach Anspruch 5, dadurch gekennzeichnet, daß die Druckmeßeinrichtung (23) zur Übertragung des gemessenen Druckistwertes und zum Vergleich des Istwertes mit dem in der Versorgungseinheit (17) speicherbaren Drucksollwert über eine Signalleitung (24) an eine Steuereinheit (25) anschließbar ist.

7. Kolben-Zylindereinheit nach Anspruch 6, dadurch gekennzeichnet, daß die Antriebsvorrichtung (7) bei fehlender Übereinstimmung zwischen Druckistwert und -sollwert durch die Steuereinheit (25) ausschaltbar ist.

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | **EINSCHLÄGIGE DOKUMENTE** | | EP 89108032.7 |
| X | US - A - 4 010 761 (N.A.TIPPLE) * Fig. 3; Spalte 5, Zeilen 1-33 * -- | 1,2 | A 61 M 16/00 |
| A | US - A - 3 741 209 (B.J.KIPLING) * Fig. 1; Spalte 2, Zeilen 1-38 * ---- | 1 | |

| | RECHERCHIERTE SACHGEBIETE (Int. Cl.4) |
|---|---|
| | A 61 M 16/00 |

Der vorliegende Recherchenbericht wurde für alle Patentansprüche erstellt.

| Recherchenort | Abschlußdatum der Recherche | Prüfer |
|---|---|---|
| WIEN | 31-08-1989 | LUDWIG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein-stimmendes Dokument

EPA Form 1503. 03 82